# EUROPEAN PATENT APPLICATION

(11) **EP 4 092 417 A1**
(43) Date of publication of application: **23.11.2022**
(21) Application number: 21740720.4
(22) Date of filing: 05.01.2021
(51) Int. Cl.: G01N 33/68

(54) **LECTIN-MACROMOLECULE CARRIER COUPLING COMPLEX FOR SEPARATING GLYCOSYLATED EXOSOME IN CLINICAL SAMPLE**

(30) Priority: 19.01.2020 CN 202010060063
(71) Applicant: Beijing Glyexo Gene Technology Co., Ltd., Daxing District, Beijing 102600 (CN)
(72) Inventor: LIN, Changqing, Beijing 102600 (CN); CHEN, Tiansheng, Beijing 102600 (CN); HAO, Kun, Beijing 102600 (CN); HUANG, He, Beijing 102600 (CN); GAO, Qi, Beijing 102600 (CN); LI, Yanzhao, Beijing 102600 (CN); XU, Zhihui, Beijing 102600 (CN); QIE, Shuang, Beijing 102600 (CN); ZHAO, Guangzhen, Beijing 102600 (CN)
(74) Representative: LLR
(86) International application number: PCT/CN2021/070289
(87) International publication number: WO 2021/143577

(57) **Abstract**

The present invention relates to a lectin-macromolecular carrier coupling complex for separating glycosylated exosomes from a clinical sample, which comprises a macromolecular carrier and lectins coupled to the outer side of the macromolecular carrier. The complex may simply, conveniently, rapidly, and accurately separate glycosylated exosomes from a clinical sample with a high separation efficiency and a good repeatability; and the separated exosomes are intact in morphology without rupturing or cracking, may be directly used for liquid detection of glycosylated exosomes, or directly used for immunology-related detection, or directly used for gene detection or analysis after extracting related nucleic acids from the exosomes.

## Description

### Cross-reference

The present invention claims the priority to Chinese Application No. 202010060063.1, entitled "Lectin-Macromolecular Carrier Coupling Complex For Separating Glycosylated Exosomes From Clinical Sample", filed in the Patent Office of the People's Republic of China, which is incorporated herein by reference in its entirety.

### Technical Field

The present invention belongs to the technical fields of biological medicines and bioseparation and relates to a lectin-macromolecular carrier coupling complex for separating glycosylated exosomes from a clinical sample.

### Background Art

Exosomes are small vesicles with biological activities, which are released when muhivesicular bodies are fused to plasma membranes, have diameters of about 30-150 nm, and are one of important media for intercellular communication. Under a normal physiological condition of human body, exosomes may deliver various bioactive substances such as DNA, proteins, mRNA, and miRNA among cells, and participate in various processes such as cell communication, cell migration, and tumor cells growth so as to complete intercellular delivery of substances and information. Literature researches have reported that nearly all types of cells can secrete the exosomes, which can be extracted from body fluids such as blood, saliva, urine, cerebrospinal fluids, hydrops, and breast milk.

Exosomes are related to many diseases including tumors, chronic infectious diseases, and autoimmune diseases. When a body is diseased, the composition, content, and properties and the like of exosomes secreted by the cells may change. Glycosylated exosomes play an important role in the occurrence and development of various diseases including tumors; and detection of exosomes can effectively monitor the occurrence and development of diseases. Due to a relatively low content of exosomes in a sample, separation and enrichment of the exosomes are crucial to researches on the exosomes.

At present, there are many separation methods for the exosomes, mainly including ultracentrifugation or density-gradient centrifugation and an immunomagnetic separation method and the like. The ultracentrifugation or the density-gradient centrifugation method conducts the separation based on small differences in density between the exosomes and other biological components, so that these methods require special equipment(s) and large amounts of samples and are time-consuming; and as different clinical samples have different characteristics, the method has a poor stability in the separation effect, and further, the ultracentrifugation method is also easy to damage vesicles, so as to affect subsequent experiment(s). The principle of the immunomagnetic separation method is that specific proteins on the surfaces of the exosomes, such as CD9, CD63, and CD81, bind to immunomagnetic beads coated with the corresponding antibodies for magnetic separation; however, the immunomagnetic beads are relatively small in size and have nano-scale particle sizes, which are smaller than or equal to the diameters of the exosomes, so that steric hindrance of the immunomagnetic beads binding to the exosomes is relatively large, causing that the binding to the exosomes is insufficient, and the separation efficiency thereof is low. In addition, the exosomes obtained by the immunomagnetic separation method are incomplete in morphology since they are eluted with an acid eluate.

Therefore, it is necessary to develop a composition, a separating device, and a separation method useful for accurately and efficiently separating and enriching glycosylated exosomes in a sample.

### Summary of the Invention

To address the deficiencies in the prior art, the present invention provides a lectin-macromolecular carrier coupling complex for separating glycosylated exosomes from a clinical sample, a composition for separating glycosylated exosomes containing the lectin-macromolecular carrier coupling complex, and a method of separating glycosylated exosomes by using the lectin-macromolecular carrier coupling complex and use thereof. The lectin-macromolecular carrier coupling complex provided by the present invention may accurately separate glycosylated exosomes from a clinical sample and achieve a high separation efficiency; and the separated exosomes are intact in morphology without rupturing or cracking, may be directly used for liquid detection of glycosylated exosomes, or directly used for immunology-related detection, or directly used for gene detection or analysis after extracting related nucleic acids from the exosomes. The method of separating glycosylated exosomes provided by the present invention takes the principle that sugar chains rich on the surfaces of the exosomes may bind to lectins, and uses effective centrifugal washing and effective elution method to separate glycosylated exosomes, which is very simple, convenient, and rapid, and has a good repeatability; and the separated exosomes are intact in morphology, without rupturing or cracking, and can be applied to detection, monitoring, and diagnosis on the occurrence, development and other processes of diseases.

The present invention provides a lectin-macromolecular carrier coupling complex for separating glycosylated exosomes from a clinical sample, which comprises a macromolecular carrier and lectins coupled to the outer side of the macromolecular carrier, wherein
the lectins are any one type of Artocarpus integrifolia lectin, peanut lectin, Pisum sativum lectin (WA and/or VVL), Concanavalin lectin, Lens culinaris lectin, wheat germ lectin, soybean lectin, kidney bean lectin, and snail lectin (HAA and/or HPA), or a combination of two or more of the above.

The macromolecular carrier is any one type of a dextran microsphere, an agarose microsphere, a resin or epoxy resin microsphere, and a polystyrene microsphere, or a combination of two or more of the above.

In some embodiments, for the lectin-macromolecular carrier coupling complex, a particle size distribution range of the macromolecular carrier is from 1 µm to 200 µm, preferably, from 10 µm to 200 µm, and more preferably, from 30 µm to 150 µm.

In some embodiments, in the lectin-macromolecular carrier coupling complex, 1-20 mg, preferably, 5-15 mg, and more preferably, 10-15 mg of lectins are coupled to each 1 mL of the macromolecular carrier.

In some embodiments, for the lectin-macromolecular carrier coupling complex, the clinical sample is any one of serum, plasma, saliva, a tissue or cell culture supernatant, urine, a cerebrospinal fluid, and a lymph fluid.

In some embodiments, for the lectin-macromolecular carrier coupling complex, the lectins are any one type of Artocarpus integrifolia lectin, peanut lectin, Pisum sativum lectin (WA and/or VVL), Concanavalin lectin, Lens culinaris lectin, wheat germ lectin, soybean lectin, and kidney bean lectin, or a combination of two or more of the above.

In some embodiments, for the lectin-macromolecular carrier coupling complex, the glycosylated exosomes are any one type of N-glycosylated exosomes, O-glycosylated exosomes, and fucosylated exosomes, or a combination of two or more of the above.

In some embodiments, the lectin-macromolecular carrier coupling complex is preserved in a preservation solution, and the preservation concentration (volume ratio) of the lectin-macromolecular carrier coupling complex in the obtained lectin-macromolecular carrier coupling complex preservation solution is 10%-60%, preferably, 30%-50%, and more preferably, 40%-50%; and the volume of the lectin-macromolecular carrier coupling complex preservation solution refers to a total volume of the lectin-macromolecular carrier coupling complex, after being dispersed in the preservation solution, and the preservation solution.

In some embodiments, for the lectin-macromolecular carrier coupling complex, the lectin-macromolecular carrier coupling complex preservation solution is loaded into a separating device comprising an affinity adsorption centrifuge tube; the affinity adsorption centrifuge tube comprises two parts which are an upper centrifuge tube and an outer protective sleeve, respectively; the diameter of the upper centrifuge tube is smaller than that of the outer protective sleeve, the upper centrifuge tube is sleeved in the outer protective sleeve, and protruding annular edges or pillars are provided on the outer side of the upper part of the upper centrifuge tube and are used for supporting an opening of the upper centrifuge tube to be higher than the outer protective sleeve; the upper centrifuge tube comprises a centrifuge tube cap, a centrifuge tube wall, and a filter membrane bottom fixedly attached to the centrifuge tube wall, in which the filter membrane of the filter membrane bottom has a pore size smaller than the particle size of the lectin-macromolecular carrier coupling complex and larger than the particle sizes of exosomes. The lectin-macromolecular carrier coupling complex preservation solution is retained in the upper centrifuge tube to form a separating device for glycosylated exosomes; and the pore size of the filter membrane is, preferably, 150-1000 nm.

In some embodiments, for the lectin-macromolecular carrier coupling complex, the volume of the lectin-macromolecular carrier coupling complex in the lectin-macromolecular carrier coupling complex preservation solution loaded into the upper centrifuge tube accounts for 1/2-1/4 of that of the upper centrifuge tube.

In another aspect, the present invention further provides a composition for separating glycosylated exosomes, comprising the lectin-macromolecular carrier coupling complex, a washing buffer for washing and for removing the exosomes nonspecifically bound to the coupling complex or not glycosylated and other impurities in the separation process, and/or an elution buffer for eluting the glycosylated exosomes specifically bound to the lectin-macromolecular carrier coupling complex. Here, the composition does not refer to mixing the above components together, but refers to co-existence of the above components in one suite at the same time.

In some embodiments, for the composition for separating glycosylated exosomes, the washing buffer is a metal salt ion-free washing buffer or purified water, and optionally, is a metal salt ion-free washing buffer, which is used for washing and for removing the exosomes nonspecifically bound to the coupling complex or not glycosylated and other impurities in the separation process. In some embodiments, for the composition for separating glycosylated exosomes, the elution buffer is a borate buffer with saccharides dissolved therein, and optionally, is a borate buffer with mannose dissolved therein; further optionally, the elution buffer has a pH value of 6.5±0.2; the elution buffer is used for eluting the glycosylated exosomes bound to the lectin-macromolecular carrier coupling complex; and the eluted exosomes are intact in morphology without rupturing or cracking.

In another aspect, the present invention further provides a method of separating glycosylated exosomes, comprising a separation step of using the above lectin-macromolecular carrier coupling complex to separate glycosylated exosomes. The separation step comprises:
pretreatment of a clinical sample:
optionally diluting the pretreated sample by mixing the same with a washing buffer uniformly to obtain a sample to be detected;
completely removing the preservation solution portion in the lectin-macromolecular carrier coupling complex preservation solution, while retaining the lectin-macromolecular carrier coupling complex;
adding the sample to be detected to the lectin-macromolecular carrier coupling complex with removal of the preservation solution, leaving the mixture to stand at room temperature for incubation, and removing the sample;
washing the lectin-macromolecular carrier coupling complex bound to the glycosylated exosomes with the washing buffer, and then removing the washing buffer for washing and for removing the exosomes nonspecifically bound to the coupling complex or not glycosylated and other impurities in the separation process; and
eluting the washed lectin-macromolecular carrier coupling complex with the elution buffer, leaving the system to stand still at room temperature, and collecting the elution supernatant, which is a solution of the separated glycosylated exosomes.

In some embodiments, for the method of separating glycosylated exosomes, a ratio of the sample to be detected to the lectin-macromolecular carrier coupling complex is 1: (1-3);
and/or a ratio of the volume of the washing buffer in a single addition to the volume of the lectin-macromolecular carrier coupling complex bound to glycosylated exosomes is (1-3): 1; and/or the washing step with the washing buffer is repeated for 1-3 times;
and/or a ratio of the volume of the added elution buffer to the volume of the washed lectin-macromolecular carrier coupling complex is (0.5-2): 1, and preferably, is (0.5-1): 1.

In some embodiments, for the method of separating glycosylated exosomes, completely removing the preservation solution portion in the lectin-macromolecular carrier coupling complex preservation solution, removing the sample, removing the washing buffer, and/or collecting the elution supernatant are all performed by centrifugation at room temperature at a speed of 3000 rpm or below for 20 s.

In some embodiments, for the method of separating glycosylated exosomes, the incubation is conducted at room temperature for 10-30 min, and preferably, for 10-15 min.

In some embodiments, for the method of separating glycosylated exosomes, the pretreatment of the sample comprises the following steps:
for a serum sample, a plasma sample, a saliva sample, a cerebrospinal fluid sample, or a lymph fluid sample, subjecting the sample to centrifugation at a speed of 3000 g or below for 10-15 min,
to remove cell debris, precipitates, and other impurities in the sample, and taking the supernatant after centrifugation for later use; and
for a tissue or cell culture supernatant sample or a urine sample, subjecting the sample to centrifugation at a speed of 3000 g or below for 10-15 min, to remove cell debris, precipitates, and
other impurities in the sample, and then concentrating the supernatant by 10-1000 times through an ultrafiltration tube for later use.

In some embodiments, for the method of separating glycosylated exosomes, the washing buffer is a metal salt ion-free washing buffer or purified water, and optionally, is a metal salt ion-free washing buffer, and further optionally, is a metal salt ion-free washing buffer with a pH value of 7.6±0.2.

In some embodiments, for the method of separating glycosylated exosomes, the elution buffer is a borate buffer with saccharides dissolved therein, and optionally, is a borate buffer with mannose dissolved therein, and further optionally, is a borate-mannose buffer with a pH value of 6.5±0.2.

In some embodiments, for the method of separating glycosylated exosomes, the incubation is conducted under the conditions of: at room temperature for 1-30 min, preferably, for 5-20 min, and more preferably, for 10-15 min.

In some embodiments, the method of separating glycosylated exosomes comprises a separation step of using the above lectin-macromolecular carrier coupling complex and the separating device to separate the glycosylated exosomes. The separation step comprises:
pretreatment of a clinical sample:
optionally diluting the pretreated sample by mixing the same with a washing buffer uniformly to obtain a sample to be detected, ;
completely removing the preservation solution portion in the lectin-macromolecular carrier coupling complex preservation solution from the affinity adsorption centrifuge tube loaded with the lectin-macromolecular carrier coupling complex, while retaining the lectin-macromolecular carrier coupling complex, and replacing the outer sleeve with a new one;
adding the sample to be detected to the upper centrifuge tube, from which the preservation solution has been removed, leaving the mixture to stand still at room temperature for incubation, and removing the sample;
adding the washing buffer to the upper centrifuge tube, taking out the upper centrifuge tube after centrifuging the affinity adsorption centrifuge tube, charging the upper centrifuge tube in the new outer sleeve, completely discarding the original outer sleeve and liquid therein for washing and for removing the exosomes nonspecifically bound to the coupling complex or not glycosylated and other impurities in the separation process, wherein this step is conducted for 1-3 times; and
adding the eluate to the upper centrifuge tube, to elute the washed lectin-macromolecular carrier coupling complex, leaving the system to stand still at room temperature, and collecting an elution supernatant, which is a solution of the separated glycosylated exosomes, from the outer sleeve, wherein the eluted glycosylated exosomes are intact in morphology without rupturing or cracking.

The present invention further provides use of exosomes separated by using the above method, comprising uses for liquid detection of glycosylated exosomes, for immunological detection of the exosomes, or for detection or analysis of nucleotide fragments after nucleic acid extraction from the exosomes.

### Beneficial effect:

1. For the lectin-macromolecular carrier coupling complex of the present invention, the exosomes obtained by separation with the coupling complex are all glycosylated exosomes with intact morphology and without rupturing, may be directly used for liquid detection of glycosylated exosomes, for immunological detection of the exosomes, or for nucleotide sequence detection or analysis after nucleic acid extraction from the exosomes, and the like, and may further achieve full-automatic separation of glycosylated exosomes in cooperation with corresponding equipments.
2. In the lectin-macromolecular carrier coupling complex of the present invention, the macromolecular carrier has a particle size of 10-1000 times that of the macromolecular carrier commonly used in the immunodetection field; and a single macromolecular carrier has a larger surface area, so that steric hindrance of the lectins being coupled to the macromolecular carrier is decreased, facilitating to couple more lectins. In contrast, although the same total volume of common macromolecular carriers has larger specific surface area, less lectins will be coupled to the macromolecular carrier due to larger steric hindrance. In the lectin-macromolecular carrier coupling complex of the present invention, 1-20 mg of lectins may be coupled to each 1 mL of macromolecular carrier; whereas researches have found that when the macromolecular carrier with a smaller volume is used, the amount of the lectins coupled to each 1 mL of macromolecular carrier is in an ng scale, indicating low coupling efficiency. Meanwhile, the particle size of the macromolecular carrier is also far larger than the diameters of the exosomes, so that steric hindrance of the lectins binding to the glycosylated exosomes may be decreased, thereby facilitating the binding of the lectins to the glycosylated exosomes, and greatly improving the separation efficiency of the exosomes.
3. The lectin-macromolecular carrier coupling complex of the present invention has good effect in separating exosomes from a clinical sample containing less exosomes, such as urine or a tissue or cell culture supernatant; and when used for separating the exosomes in a clinical sample containing more exosomes, such as plasma, the lectin-macromolecular carrier coupling complex can separate a concentration of up to 10¹¹⁻¹⁴/mL of the glycosylated exosomes.
4. In the lectin-macromolecular carrier coupling complex of the present invention, the used lectins are preferably phytolectins, including Artocarpus integrifolia lectin, peanut lectin, Pisum sativum lectin (WA and/or VVL), Concanavalin lectin, Lens culinaris lectin, wheat germ lectin, soybean lectin, and kidney bean lectin; and the phytolectins are more easily available in a great amount and raw materials are more abundant.
5. The lectin-macromolecular carrier coupling complex of the present invention may be loaded into the affinity adsorption centrifuge tube of a specific structure to form a separating device for the glycosylated exosomes as leaving factory, which facilitates use of a user and also preservation of the lectin-macromolecular carrier coupling complex. The selected pore size of the filter membrane facilitates smooth passing of various solutions such as the preservation solution, the washing buffer, and the eluate solution; particularly, due to the addition of mannose, the viscosity of the elution buffer is increased; and with the selected pore size, smooth passing of the elution buffer and no leakage of the lectin-macromolecular carrier coupling complex can both be ensured.
6. For the composition for separating glycosylated exosomes according to the present invention, the metal salt ion-free washing buffer or purified water is used; metal salt ions have a dissociation effect on affinity adsorption between the lectins and the glycosylated exosomes, which affects the affinity adsorption between the lectins and the glycosylated exosomes; and in addition, the higher the concentration of the metal salt ions is, the stronger the dissociation effect is, so that the separation effect of the exosomes is impaired, and even that the exosomes cannot be separated.
7. For the composition for separating glycosylated exosomes according to the present invention, the used elution buffer is the borate buffer; compared with other buffers, the borate buffer has better elution effect on the exosomes, exhibiting high elution efficiency, and elution may be accomplished with a small amount of the elution buffer; the eluted exosomes are intact in morphology without rupturing or cracking; and meanwhile, due to less usage amount, concentration of exosomes may be accomplished at the same time.
8. For the method of separating glycosylated exosomes according to the present invention, the pretreatment of the sample is simple and feasible; and the sample suitable for the present invention may be effectively obtained through simple centrifugation and/or concentration without centrifuging the sample at an ultra high speed, which may protect the glycosylated exosomes in the sample to the greatest extent.

### Brief Description of the Drawings

FIG. 1 is a schematic diagram of an affinity adsorption centrifuge tube of a lectin-macromolecular carrier coupling complex.
FIG. 2 is a set of electron microscopic images showing the separation of glycosylated exosomes from 4 different types of samples (cell culture supernatant, plasma, urine, and cerebrospinal fluid).
FIG. 3 is a NTA analysis diagram of glycosylated exosomes separated from a plasma sample.
FIG. 4 is a diagram showing results of Western Blot analysis of exosomes separated from 4 different types of samples (cell culture supernatant, plasma, urine, and cerebrospinal fluid) with antibodies against exosome-specific membrane proteins CD9, CD63, and CD81.
FIG. 5 is a diagram showing results of Western Blot analysis of exosomes separated from 4 different types of samples (cell culture supernatant, plasma, urine, and cerebrospinal fluid) with antibodies against exosome-specific non-membrane proteins ALIX and TSG101.
FIG. 6 is a diagram showing results of Western Blot analysis of exosomes separated from 4 different types of samples (cell culture supernatant, plasma, urine, and cerebrospinal fluid) with an anti-Calnexin antibody.
FIG. 7 is a diagram showing results of Western Blot analysis of eluates with antibodies against exosome-specific membrane proteins CD9, CD63, and CD81, wherein the eluates were obtained by washing the lectin-macromolecular carrier coupling complexes bound to glycosylated exosomes with 5 different washing buffers, respectively, and then eluting the same.
FIG. 8 is a set of electron microscopic images of solutions of glycosylated exosomes separated by 3 different elution buffers, respectively.

### Detailed Description of the Invention

In order to better describe the present invention, various illustrative embodiments of the present invention are described in detail now. The details description should not be considered as limiting to the present invention and should be understood as more detailed description of certain aspects, features, and embodiments of the present invention.

It should be apparent to those skilled in the art that various improvements and variations may be made to the specific embodiments of the description of the present invention without departing from the scope or spirit of the present invention. The description and the embodiments of this application are illustrative merely.

Terms "comprising", "including", "having", "containing" and the like used herein are open terms, that is, the terms mean including, but not limited to. Unless expressly specified otherwise, various reagents used below are all commercial reagents, wherein used chemical reagents are analytically pure.

### Example 1. Preparation of Lectin-Macromolecular Carrier Coupling Complex

A lectin-macromolecular carrier coupling complex comprises a macromolecular carrier, and lectins coupled to the outer side of the macromolecular carrier. The lectin-macromolecular carrier coupling complex is prepared by coupling the lectins to the macromolecular carrier under specific conditions and is mainly used for separating glycosylated exosomes in a sample. The glycosylated exosomes obtained by the separation are intact in morphology, wherein:
the lectins are mainly selected from any one of Artocarpus integrifolia lectin (Jacalin), peanut lectin (PNA), Pisum sativum lectin (VVA and/or VVL), Concanavalin lectin (ConA), Lens culinaris lectin (LCA), wheat germ lectin (WGA), soybean lectin (SBA), kidney bean lectin (PVL), and snail lectin (HAA and/or HPA), or two or more of the above lectins used in combination; the main reason for selecting two or more lectins used in combination is: this combination of different lectins may achieve separation of various types of glycosylated exosomes, such as separation of fucosylated exosomes with LCA and AAL, separation of N-glycosylated exosomes with lectins such as ConA, PVL, SBA, WGA, or AAL, and separation of O-glycosylated exosomes with lectins such as HAA, HPA, VVA, PNA, or Jacalin; and a single type of lectins are mainly used for separating a specific type of glycosylated exosomes corresponding to the lectins, whereas two or more of the lectins used in combination may be used for separating glycosylated exosomes in various forms, or two or more of the lectins used in combination may be used for synergistically separating the specific glycosylated exosomes.

For further illustration of the present invention, the following examples mainly use the Lens culinaris lectin (LCA) (purchased from Sigma Company, America) for further description of the present invention.

The macromolecular carrier mainly refers to a macromolecular microsphere and is mainly selected from any one of a dextran microsphere, an agarose microsphere, a resin or epoxy resin microsphere, and a polystyrene microsphere, or two or more of the above macromolecular carriers used in combination. A particle size distribution range of the macromolecular carriers is from 1 µm to 200 µm, preferably, from 10 µm to 200 µm, and more preferably, from 30 µm to 150 µm. In the production of the macromolecular carriers, the particle sizes of the macromolecular carriers produced in the same batch are usually not uniform. Therefore, the particle sizes of the macromolecular carriers may be usually described either in the average particle size, or in a particle size distribution range as adopted in the present invention. For the description manner in the particle size distribution range, it may be considered that the specific particle size distribution conforms to or substantially conforms to the normal distribution within this distribution range. In the lectin-macromolecular carrier coupling complex of the present invention, the particle size of the used macromolecular carrier is 10-1000 times that of the macromolecular carrier commonly used in the immunodetection field; and a single macromolecular carrier has a larger surface area, so that steric hindrance of the lectins being coupled to the macromolecular carrier is decreased, facilitating to couple more lectins. In contrast, although the same total volume of common macromolecular carriers has larger specific surface area, less lectins are coupled to the macromolecular carrier due to larger steric hindrance. In the lectin-macromolecular carrier coupling complex of the present invention, 1-20 mg of lectins may be coupled to each 1 mL of macromolecular carrier; whereas researches have found that when the macromolecular carrier with a smaller volume is used, the amount of the lectins coupled to each 1 mL of macromolecular carrier is in an ng scale, indicating low coupling efficiency. Meanwhile, the particle size of the macromolecular carrier is also far larger than the diameters of the exosomes, so that steric hindrance of the lectins binding to the glycosylated exosomes may be decreased, thereby facilitating the binding of the lectins to the glycosylated exosomes, and greatly improving the separation efficiency of the exosomes.

The lectins are coupled to the macromolecular carrier to form the lectin-macromolecular carrier coupling complex. Different types of macromolecular carriers can all achieve the effect of coupling to the lectins by adjusting steps of soaking, coupling, washing, preservation, and the like. Certainly, for different types of lectins, the most suitable macromolecular carriers may be different. In the preparation process of the lectin-macromolecular carrier coupling complex, a ratio of the macromolecular carrier (mL) to the lectins (mg) is (1:1)-(1:20), preferably, is (1:5)-(1:15), and more preferably, is (1:10)-(1:15).

The prepared lectin-macromolecular carrier coupling complex is preserved in a preservation solution; and the preservation concentration (volume ratio) of the lectin-macromolecular carrier coupling complex in the obtained lectin-macromolecular carrier coupling complex preservation solution is 10%-60%, preferably, is 30%-50%, and more preferably, is 40%-50%. The lectin-macromolecular carrier coupling complex preservation solution is loaded into an upper centrifuge tube of an affinity adsorption centrifuge tube. The affinity adsorption centrifuge tube comprises two parts which are an upper centrifuge tube and an outer protective sleeve, respectively; the diameter of the upper centrifuge tube is smaller than that of the outer protective sleeve, the upper centrifuge tube is sleeved in the outer protective sleeve, and protruding annular edges or pillars are provided on the outer side of the upper part of the upper centrifuge tube and are used for supporting an opening of the upper centrifuge tube to be higher than the outer protective sleeve; the upper centrifuge tube comprises a centrifuge tube cap, a centrifuge tube wall, and a filter membrane bottom fixedly attached to the centrifuge tube wall, in which the filter membrane of the filter membrane bottom has a pore size smaller than the particle size of the lectin-macromolecular carrier coupling complex and larger than particle sizes of the exosomes, which is 150-1000 nm preferably; and the upper centrifuge tube is used for charging the lectin-macromolecular carrier coupling complex to form a separating device for glycosylated exosomes.

For further description of the present invention, the following examples all employ agarose microspheres with a particle size distribution range from 30 µm to 150 µm. The agarose microspheres are commercial hydrogen bromide-activated agarose microspheres (purchased from Pharmacia Company or Sigma Company). The agarose microspheres are, preferably, sephrose 4B, sephrose 6B, sephrose FF, sephrose CL-4B, or sephrose CL-6B. The following example takes sephrose 4B as an example for illustration.

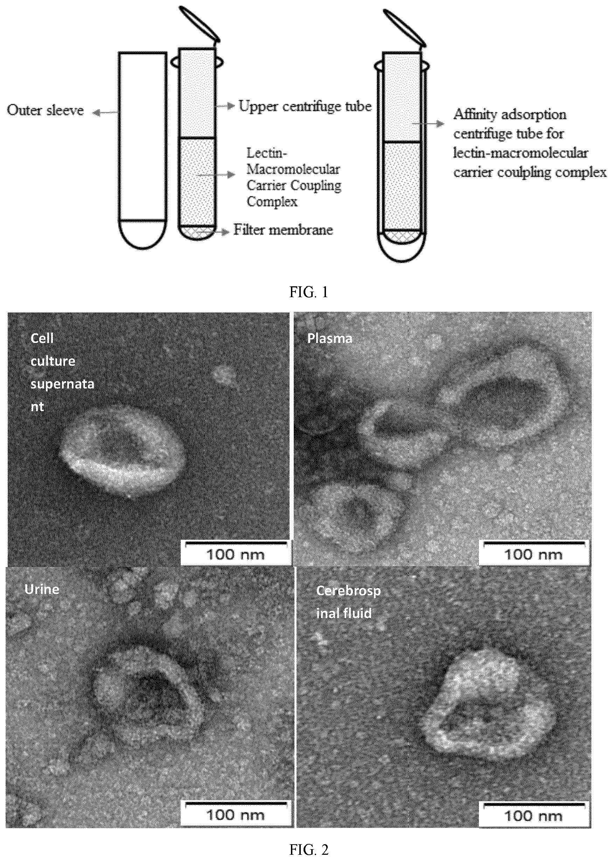

**The preparation process of the lectin-macromolecular carrier coupling complex, i.e. LCA-agarose microsphere coupling complex was as follows in detail:**
(1) To 1 g of activated sephrose 4B, no less than 200 mL of 1 mM HCl solution with a pH value of 2-3 was added for uniform mixing; the activated sephrose 4B was soaked until being completely swollen; the swollen sephrose was washed for 15 min or more with 1 mM HCl solution with the pH value of 2-3 in a glass fritted funnel; after washing was completed, the completely swollen sephrose 4B was collected for later use, wherein the volume of the completely swelled sephrose 4B was about 3-5 mL.
(2) To 15 mL of 0.1-1.0 M carbonate buffer solution, Lens culinaris lectin was added, and then the completely swollen sephrose 4B collected in the step (1) was added, the completely swollen sephrose 4B and the Lens culinaris lectin were uniformly mixed for reaction for 0.5-5 h at room temperature, and then a supernatant was removed, wherein a ratio of the volume (mL) of the completely swollen sephrose 4B to the weight of the Lens culinaris lectin (LCA, mg) was (1:1)-(1:20) (that is, each 1 mL of the completely swollen sephrose 4B being mixed with 1-20 mg of Lens culinaris lectin). For better description of the present invention, in this example, the working concentration of the carbonate buffer solution was 0.1 M, and the ratio of the volume (mL) of the completely swollen sephrose 4B to the weight of the Lens culinaris lectin (LCA, mg) was 1:10, that is, 5 mL of the completedly swollen sephrose 4B and 50 mg of LCA were added to 15 mL of the carbonate buffer solution for uniform mixing and reaction for 3 h at room temperature.
(3) 100 mL of 0.1 M the carbonate buffer was added for uniform mixing, and a supernatant was removed; and then 200 mL of purified water was added for uniform mixing, and a supernatant was removed.
(4) The precipitate was repeatedly washed by not less than 10 times volume of the completely swollen sephrose 4B of 0.1 mol/L acetate buffer (containing 0.5 mol/L NaCl) with a pH value of 4.0 and 0.1 mol/L Tris-HCl buffer (containing 0.5 mol/L NaCl) with a pH value of 8.0 in sequence for at least 3 times.
(5) 0.1 mol/L TRIS-HCl buffer with a pH value of 8.0 was added for washing for one time, followed by collecting the completely swollen sephrose 4B, to which the TRIS-HCl buffer was added to prepare 10%-60%, preferably, 30%-50% of LCA-agarose microsphere preservation solution, for later use. For better description of the present invention, in this example, a volume ratio of 50% of the LCA-agarose microsphere preservation solution was prepared.
(6) The 50% of LCA-agarose bead preservation solution prepared in the step (5) was mixed uniformly and loaded into a separating device comprising an affinity adsorption centrifuge tube to form a separating device for glycosylated exosomes, which was sealed and allowed to stand still, and was preserved at 2-8°C for later use. The affinity adsorption centrifuge tube comprised two parts which were an upper centrifuge tube and an outer protective sleeve, respectively; the diameter of the upper centrifuge tube was smaller than that of the outer sleeve, and protruding annular edges or pillars were provided on the outer side of the upper part of the upper centrifuge tube and were used for supporting an opening of the upper centrifuge tube to be higher than the outer sleeve; the upper centrifuge tube comprised a centrifuge tube cap, a centrifuge tube wall, and a filter membrane bottom fixedly attached to the centrifuge tube wall, in which the filter membrane of the filter membrane bottom had a pore size smaller than the particle size of the lectin-macromolecular carrier coupling complex and larger than the particle sizes of the exosomes. The lectin-macromolecular carrier coupling complex preservation solution was retained in the upper centrifuge tube; and optionally, the pore size of the filter membrane was 150-1000 nm. The size of the upper centrifuge tube of the affinity adsorption centrifuge tube may be designed according to the size of specific centrifugal equipment; the volume of the lectin-macromolecular carrier coupling complex accounted for 1/2-1/4 of that of the upper centrifuge tube; and if 1.5-2 mL upper centrifuge tube was selected, the lectin-macromolecular carrier coupling complex preservation solution containing 0.5 mL of lectin-macromolecular carrier coupling complex was loaded into the tube. The schematic diagram of the affinity adsorption centrifuge tube was shown in FIG. 1 in detail. The preservation solution portion in the lectin-macromolecular carrier coupling complex preservation solution could enter the outer protective sleeve through the filter membrane and was reserved in the outer protective sleeve without losing. Also, due to the sleeving structure of the upper centrifuge tube and the outer protective sleeve and the selection of volume concentration in the lectin-macromolecular carrier coupling complex preservation solution, the lectin-macromolecular carrier coupling complex could be in contact with enough of the preservation solution without drying or losing the activity.

### Example 2. Preparation of Composition for Separating Glycosylated Exosomes

The present invention provides a composition for separating glycosylated exosomes, specifically comprising: the affinity adsorption centrifuge tube (i.e. the separating device for glycosylated exosomes), in which the LCA-agarose microsphere preservation solution obtained in Example 1 was charged, a washing buffer, and an elution buffer, all of which were separately packaged and existed in one suite or kit at the same time.

The washing buffer was a metal salt ion-free washing buffer or purified water, and optionally, was a metal salt ion-free washing buffer, for example, 10-200 mM metal salt ion-free TRIS-HCl buffer with a pH value of 7.6±0.2. In this example, the washing buffer was the metal salt ion-free washing buffer, for example, 100 mM metal salt ion-free TRIS-HCl buffer with a pH value of 7.6±0.2; and the washing buffer was used for washing and for removing the exosomes nonspecifically bound to the coupling complex or not glycosylated and other impurities in the separation process.

The elution buffer was a borate buffer with saccharides dissolved therein, for example, 10-20 mM borate buffer with 100-500 mM mannose dissolved therein and with a pH value of 6.5±0.2. In this example, the borate buffer with saccharides dissolved therein was 15 mM borate buffer with 300 mM mannose dissolved therein and with a pH value of 6.5±0.2; the borate buffer was used for eluting the glycosylated exosomes bound to the lectin-macromolecular carrier coupling complex; and the eluted exosomes were intact in morphology without rupturing or cracking.

### Example 3. Use Method of the Composition for Separating Glycosylated Exosomes

The present invention further provides a method for separating glycosylated exosomes, comprising the experimental step of using the composition for separating glycosylated exosomes, which comprises:
1. Preparation before experiment
   Autonomous preparation of apparatuses or equipment: a centrifugal machine, used for centrifugation steps in the process of separating glycosylated exosomes; or a full-automatic agarose microsphere separating instrument from the Group Company and subsidiaries, mainly used for fully automatically separating the glycosylated exosomes to achieve the purpose of saving manpower. Full-automatic separation of the glycosylated exosomes with the agarose microsphere is just an automatic implementation for a manual mode and can achieve an effect equivalent to or superior to manual separation. This example employs the centrifugal machine with manual separation for further illustration.
2. Experimental procedure
   (1) pretreatment of a sample: a method of pretreatment of the sample can be adjusted according to the characteristics of different clinical samples.
      For a serum sample, a plasma sample, a saliva sample, a cerebrospinal fluid sample, or a lymph fluid sample, the sample was subjected to centrifugation at 3000 g for 10-15 min to remove cell debris, precipitates, and other impurities in the sample, and the supernatant after centrifugation was taken for later use. For further clear illustration, in this example, centrifugation was conducted for 10 min.
      For a tissue or cell culture supernatant sample or a urine sample, the sample was subjected to centrifugation at 3000 g for 10-15 min to remove cell debris, precipitates, and other impurities in the sample, and then the supernatant was concentrated by 10-1000 times through an ultrafiltration tube for later use. For further clear illustration, in this example, centrifugation was conducted for 10 min.
   (2) 200-300 uL of the pretreated sample obtained in the step (1) could not only be directly mixed with the lectin-macromolecular carrier coupling complex without being diluted with the washing buffer, but also be mixed with the lectin-macromolecular carrier coupling complex after being diluted with 1-2 times by volume of the washing buffer, wherein the effect of the latter was better. In this example, the volume of the upper centrifuge tube of the affinity adsorption centrifuge tube was 2.0 mL, and 1 mL/tube of lectin-macromolecular carrier coupling complex preservation solution (containing 0.5 mL/tube of lectin-macromolecular carrier coupling complex) was loaded into the upper centrifuge tube; the volume of the pretreated sample was selected as 200 uL, the volume of the washing buffer was 300 uL, and the volume of the pretreated sample after being diluted with the washing buffer was identical to that of the lectin-macromolecular carrier coupling complex in the affinity adsorption centrifuge tube; and the volume of the sample after being diluted with the washing buffer cannot be larger than that of the lectin-macromolecular carrier coupling complex in the affinity adsorption centrifuge tube significantly, and when the volumes of the above two were substantially the same, the binding effect was the best.
   (3) the upper centrifuge tube of the affinity adsorption centrifuge tube loaded with the lectin-macromolecular carrier coupling complex was placed into a new outer sleeve; centrifugation was conducted at 3000 rpm at room temperature for 20 s to remove the preservation solution portion from the lectin-macromolecular carrier coupling complex preservation solution, while retaining the lectin-macromolecular carrier coupling complex; and the outer sleeve was replaced with a new one.
   (4) 500 uL of the diluted sample obtained in the step (2) was added to the upper centrifuge tube, leaving it to stand still for incubation for 10-30 min, preferably, 10-15 min, and then the affinity adsorption centrifuge tube was subjected to centrifugation at 3000 rpm at room temperature for 20 s to remove the sample. In this example, the incubation time was selected as 10 min.
   (5) 500-1500 uL of 100 mM TRIS-HCl buffer with a pH value of 7.6±0.2, serving as the metal salt ion-free washing buffer, was added to the upper centrifuge tube; the affinity adsorption centrifuge tube was subjected to centrifugation at 3000 rpm at room temperature for 20 s; the upper centrifuge tube was taken out and placed into a new outer sleeve; the original outer sleeve and liquid therein were completely discarded for washing and for removing the exosomes nonspecifically bound to the coupling complex or not glycosylated and other impurities in the separation process; and the step (5) was conducted for 1-3 times. In this example, the volume of the salt ion-free washing buffer was 1 mL.
   (6) A borate-mannose buffer, with the volume the same as that of the lectin-macromolecular carrier coupling complex in the affinity adsorption centrifuge tube and with a pH value of 6.5±0.2, was added to the upper centrifuge tube, as the elution buffer, wherein the elution buffer comprised 15 mM borate buffer and 300 mM mannose dissolved therein; the washed lectin-macromolecular carrier coupling complex was eluted, leaving it to stand still at room temperature for 5-10 min; centrifugation was conducted at 3000 rpm at room temperature for 20 s; and the elution supernatant, which was a solution of the separated glycosylated exosomes, in the outer sleeve was collected, wherein the glycosylated exosomes separated by elution were intact in morphology without rupturing or cracking. In this example, the volume of the elution buffer was 500 uL; the elution buffer was added in the upper centrifuge tube, which was then covered by a centrifuge tube cap, allowed to stand still at room temperature for 5 min, and then centrifuged at 3000 rpm at room temperature for 20 s; and the elution supernatant, which was a solution of the separated glycosylated exosomes, in the outer sleeve was collected and was directly used for detection or preserved at -80±5 °C for later use.

### Example 4

This example mainly makes further illustration for samples from which glycosylated exosomes are to be separated. Samples which can be used for the present invention may be selected from any one of serum, plasma, saliva, a tissue or cell culture supernatant, urine, a cerebrospinal fluid, and a lymph fluid. For a conventional 1.5-2 mL upper centrifuge tube, 1 mL/tube lectin-macromolecular carrier coupling complex preservation solution (containing 0.1-0.6 mL/tube lectin-macromolecular carrier coupling complex, for example, 0.5 mL) may be loaded thereinto; and for 600 uL of lectin-macromolecular carrier coupling complex, 50-600 uL, preferably, 100-300 uL, more preferably, 200-300 uL of the pretreated sample of the serum, the plasma, the saliva, the cerebrospinal fluid, the lymph fluid, the tissue or cell culture supernatant, or the urine may be added thereto, and the sample may be directly added, or be added after being diluted with the washing buffer. In the pretreatment process of the sample, the serum sample, the plasma sample, the saliva sample, the cerebrospinal fluid sample, or the lymph fluid sample was subjected to centrifugation only, and there was no change substantially in volume of the sample before and after centrifugation. Therefore, the desired volume of the pretreated sample might be obtained by pretreating 50-600 uL, preferably, 100-300 uL, more preferably, 200-300 uL of the sample; and whereas in the pretreatment process of the tissue or cell culture supernatant sample or the urine sample, in addition to the centrifugation step, it was required to concentrate the sample by 10-1000 times, and therefore, in order to obtain sufficient amount of the pretreated sample, it was required to subject 1-50 mL, preferably, 10-50 mL, more preferably, 30-50 mL of the tissue or cell culture supernatant sample or the urine sample to the pretreatment including centrifugation and concentration, upon which 50-600 uL, preferably, 100-300 uL, more preferably, 200-300 uL of the pretreated tissue or cell culture supernatant sample or the pretreated urine sample could be obtained.

Different volumes of the sample to be separated have little effect on the separation effect, but can all achieve the effect of separating glycosylated exosomes substantially. For further illustration of the present invention, in this example, according to the separation step in Example 3, 200 uL of the pretreated serum sample, the pretreated plasma sample, the pretreated saliva sample, the pretreated cerebrospinal fluid sample, or the pretreated lymph fluid sample was selected for illustration; and 50 mL of the sample to be separated and 200 uL of the pretreated sample upon concentration were selected for illustration of the tissue or cell culture supernatant sample or the urine sample.

According to the specificities of the to-be-separated samples of serum, plasma, saliva, tissue or cell culture supernatant, urine, cerebrospinal fluid, lymph fluid or the like, 4 types of samples, including the cell culture supernatant sample, the plasma sample, the urine sample, and the cerebrospinal fluid sample, were selected for further illustration; and for other types of samples than the above ones, the same separation effect can also be achieved.

According to the procedure in Example 3, glycosylated exosomes were separated from 200 uL of the pretreated cell culture supernatant sample, plasma sample, urine sample, and cerebrospinal fluid sample, respectively, in this example to obtain solutions of glycosylated exosomes separated from 4 different samples for later use.

### Example 5

In this example, the glycosylated exosomes separated in Example 4 were subjected to detection by a transmission electron microscope for the morphology and the particle sizes thereof, mainly comprising the steps of:
about 5 uL of the sample solution of glycosylated exosomes separated in Example 4 was dripped onto a copper net for still standing for 4-5 min; excess liquid was sucked off by filter paper from the edge of the copper net; a negative staining fluid (0.5% aqueous uranium acetate solution, pH 4.5) was dripped onto the copper net for reaction thereon for 1 min; the copper net was sucked to dry by the filter paper, was repeatedly washed for 2 times and dried in the air, and then was subjected to observation through Tecnai Spirit (120kV TEM) transmission electron microscope.

The observations showed that the glycosylated exosomes separated from the four different types of samples in Example 4 were intact in morphology without rupturing and had the particle sizes ranging from 30 nm to 150 nm. For more details, please see the set of electron microscopic images showing the separation of glycosylated exosomes from 4 different types of samples in Figure 2.

### Example 6

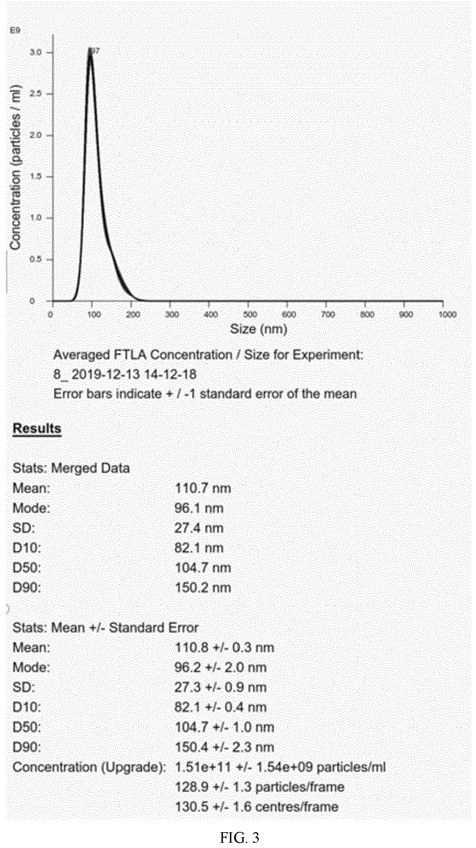

In this example, the glycosylated exosomes separated in Example 4 were subjected to nanoparticle tracking analysis (NTA) by using a NanoSight NS300 system for the granularity and particle sizes thereof; NTA is a test mainly in a solution state (in-situ test), and with its high-precision granularity number distribution test, particles with a relative particle size difference of about 1:1.5 times may be distinguished, which enables exosome particles to be measured in a state closer to an original state, and thereby ensures the authenticity and validity of the measurement; reliable concentration data of the exosomes is provided; the defect that the obtained particle size distribution data is often not representative as a range which can be observed at one time is limited when the exosomes are directly observed and measured by the electron microscope at present is effectively remedied; and damages, observed by the electron microscope, on the exosomes due to pretreatments such as drying, immobilization, freezing, and the like can be effectively lowered. Based on the consistency of results, as observed in Example 5, of 4 different types of samples, in this example, the glycosylated exosomes separated from the plasma sample were selected for NTA detection, comprising the detailed steps of: about 10 uL of sample was diluted to 1 mL; the diluted sample was loaded by a NanoSight injection pump, and was subjected to automatic analysis by the NanoSight NS300 system to obtain the results that the glycosylated exosomes were relatively uniform, had the average particle size ranging from 30 nm to 150 nm, conforming to an average particle size range of the exosomes, and had the granularity of 10¹¹-10¹⁴/mL. For more details, please see Figure 3, a NTA analysis diagram of glycosylated exosomes separated from a plasma sample.

### Example 7

In this example, the glycosylated exosomes separated in Example 4 were subjected to Western Blot analysis with antibodies against exosome-specific membrane proteins CD9, CD63, and CD81, against exosome-specific non-membrane proteins ALIX and TSG101, and against the protein Calnexin, mainly comprising the following detection steps of:
(1) preparation of a sample: 30 uL of glycosylated exosome eluate was added to an equal volume of 5×SDS-PAGE loading buffer, and the mixture was treated at 100°C for 10 min for later use.
(2) SDS electrophoresis: a gel (15% separation gel, 5% spacer gel) was prepared, 1×electrophoresis buffer was added, 10 µL of sample was added to each hole, and the electrophoresis conditions of 60 V and 120 min were set.
(3) Membrane transfer: a PVDF membrane with a suitable size was placed in a transfer buffer and soaked for 10 min, the gel and the PVDF membrane were closely adhered to a sponge pad to prevent production of bubbles, and proteins were transferred onto the PVDF membrane by using a semi-dry transfer unit (membrane transfer was conducted for 20 min at a constant voltage of 40 V and a constant current of 200 mA).
(4) Blocking: the PVDF membrane was blocked with 5% skim milk powder for 1 h, and then was washed with TBST for 3 times for 5 min each time.
(5) Incubation: the blocked PVDF membrane was incubated for 1 h with primary antibodies (an anti-CD9 monoclonal antibody, an anti-CD81 monoclonal antibody, an anti-CD63 monoclonal antibody, an anti-TSG101 monoclonal antibody, an anti-Alix monoclonal antibody, and an anti-Calnexin monoclonal antibody, with a concentration of 1 mg/mL, and being diluted by 1:400), respectively , followed by a washing step with TBST for 3 times for 5 min each time, and then was incubated with a second antibody (a goat-anti-mouse HRP, 1 mg/mL, diluted by 1:5000) for 1 h, followed by a washing step with TBST for 3 times for 5 min each time.
(6) Development: BeyoECL Moon (very sensitive ECL chemiluminescence kit) was used for color development, and then detection was conducted.

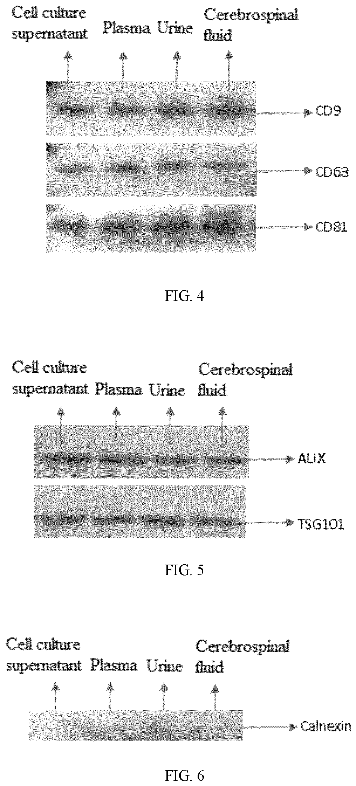

Detection results of the 4 types of glycosylated exosomes separated in Example 4 by Western Blot were as follows:
(1) The Western Blot detections by using antibodies against exosome-specific membrane proteins CD9, CD63, and CD81 were positive, indicating the presence of the exosome-specific membrane proteins CD9, CD63, and CD81. For details, please see FIG. 4: a diagram showing results of Western Blot analysis by using the antibodies against the exosome-specific membrane proteins CD9, CD63, and CD81.
(2) The Western Blot detections by using antibodies against exosome-specific non-membrane proteins ALIX and TSG101 were positive, indicating the presence of the exosome-specific non-membrane proteins ALIX and TSG10. For details, please see FIG. 5: a diagram showing results of Western Blot analysis by using the antibodies against the exosome-specific non-membrane proteins ALIX and TSG101.
(3) The Western Blot detection of exosome samples by using an anti-Calnexin antibody was negative. For details, please see FIG. 6: a diagram showing result of Western Blot analysis of exosomes by using an anti-Calnexin antibody. The protein Calnexin exists in an endoplasmic reticulum, but does not exist in exosomes. The glycosylated exosomes separated from the samples in this example were detected to be negative by Western Blot analysis using an anti-Calnexin antibody, indicating that no cell debris existed in the separated glycosylated exosomes.

### Example 8

In this example, the washing and separation effects of different washing buffers, particularly, common washing buffers containing metal salt ions, common washing buffers without metal salt ions, and purified water, in the glycosylated exosome separation process were further compared and verified, so as to determine the suitability of the selected washing buffer in the present invention.

The metal salt ion-free washing buffer (Tris-HCL) in the composition for separating glycosylated exosomes in Examples 2 and 3 was replaced by the purified water and by common washing buffers containing metal salt ions, respectively. The common washing buffers containing metal salt ions comprised: a phosphate buffer (PBS buffer), a Tris-Triton-NaCl buffer, and a TBST buffer. As most of the common washing buffers containing metal salt ions contain surfactants such as Tween-20 and Triton X-100, which can damage outer membranes of the exosome vesicles, when using the common washing buffers containing metal salt ions, the component surfactants was excluded therefrom.

In this example, the phosphate buffer (PBS buffer), the Tris-Triton-NaCl buffer without Triton X-100, and the TBST buffer without Tween-20 were respectively used as the common washing buffers containing metal salt ions.

The PBS buffer comprises the following components: 0.24 g/L of potassium dihydrogen phosphate (KH₂PO4), 1.44 g/L of disodium hydrogen phosphate (Na₂HPO4), 8 g/L of sodium chloride (NaCl), and 0.2 g/L of potassium chloride (KCl), and has a pH value of 7.4±0.2.

The above Tris-Triton-NaCl buffer without Triton X-100 comprises the following components: 50 mM Tris-HCL and 0.6 M NaCL, and has a pH value of 7.6±0.2.

The above TBST buffer without Tween-20 comprises the following components: 10 mM Tris-HCL and 0.15 M NaCL, and has a pH value of 7.6±0.2.

In this example, 5 types of washing buffers including the purified water, the Tris-Triton-NaCl buffer, the PBS buffer, the Tris-Triton-NaCl buffer without Triton X-100, and the TBST buffer without Tween-20 were used, and were marked as Washing Buffer No. 1, Washing Buffer No. 2, Washing Buffer No. 3, Washing Buffer No. 4, and Washing Buffer No. 5, respectively. According to the method in Example 3, the 5 types of washing buffers were applied in this example to separate the glycosylated exosomes in a plasma sample, with comparison and verification. In this example, except for the washing buffers, other components and steps are the same. Other detailed processes may refer to Examples 1-4, and the detection and identification steps for the exosomes by using antibodies against the exosome-specific membrane proteins CD9, CD63, and CD81 through Western Blot analysis in Example 7.

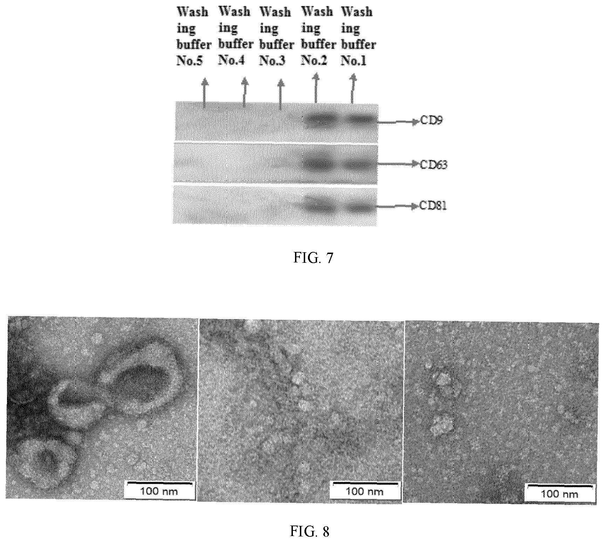

In this example, Western Blot detection results for the glycosylated exosome solutions by using the antibodies against the exosome-specific membrane proteins CD9, CD63, and CD81 were shown in FIG. 7 in detail, wherein the glycosylated exosome solutions were obtained by washing the complexes carrying glycosylated exosomes with the 5 different types of washing buffers and then eluting the same for separation.

Western Blot detection results for eluates, obtained by washing lectin-macromolecular carrier coupling complexes bounded to the glycosylated exosomes with the metal salt ion-free washing buffers (the Washing Buffer No. 1 and the Washing Buffer No. 2) and then eluting the same, show that the exosome-specific membrane proteins CD9, CD63, and CD81 are all positive, indicating that the glycosylated exosomes can be effectively separated with the both metal salt ion-free washing buffers; whereas Western Blot results for eluates, obtained by washing the lectin-macromolecular carrier coupling complexes bounded to the glycosylated exosomes with the common washing buffers containing metal salt ions but without the surfactants (the Washing Buffer No. 3, the Washing Buffer No. 4, and the Washing Buffer No. 5) and then eluting the same, showed that the exosome-specific membrane proteins CD9, CD63, and CD81 were all negative, indicating that the metal salt ions in the buffers may affect the separation effect of the glycosylated exosomes and have obvious dissociation effect on the glycosylated exosomes in the present invention.

### Example 9

In this example, the effects of separating glycosylated exosomes by different types of common protein elution buffers and a borate-mannose buffer in the present invention were compared and verified, so as to determine the suitability of the selected elution buffers in the present invention. The borate-mannose buffer in the composition for separating glycosylated exosomes in Examples 2 and 3 was replaced by a common protein and glycoprotein elution buffer, which included specifically, protein antibody elution buffer (stripping buffer) and glycoprotein elution buffer (Glycoprotein Eluting Solution) (Vectorlabs Company, America).

The above stripping buffer comprises the following components: 62.5 mmol/L Tris HCl, 2% SDS, and 100 mmol/L β-2-mercaptoethanol, and has a pH value of 6.8±0.2.

The 3 types of elution buffers were applied in this example to separate the glycosylated exosomes in a plasma sample, with comparison and verification. In this example, except for the elution buffer, other components and steps are the same. The detailed processes may refer to Examples 1-5; and the morphology and the integrity of the exosomes of the glycosylated exosomes separated from the plasma sample by the 3 types of elution buffer s were observed through an electron microscope.

In this example, electron microscopic detection results of the glycosylated exosomes separated by using 3 different types of elution buffers were shown in FIG. 8 in detail.

Results show that the elution buffers in the present invention were able to effectively separate the glycosylated exosomes in a sample, and the separated exosomes were intact in morphology without rupturing and had particle sizes of 30-150 nm; with the stripping buffer as the elution buffer, no exosome was observed, the reason for which may be that the stripping buffer, serving as the elution buffer, may damage outer membranes of the exosomes in the elution process, and thereby no intact exosomes can be obtained; whereas by using the glycoprotein elution buffer (Glycoprotein Eluting Solution), no typcial exosome vesicle was observed, only exosome-like vesicles can be observed, but the morphology thereofwas not intact, indicating that the glycoprotein elution buffer (Glycoprotein Eluting Solution) causes certain damages on the exosomes in the separation process, which goes against subsequent detection or analysis.

To sum up, by implementing the technical solutions of the present invention to separate glycosylated exosomes from different samples, the same effect can be achieved; in addition, the exosomes obtained by the separation are all glycosylated exosomes, and the exosomes are intact in morphology without rupturing after being separated and purified, and may be directly used for liquid detection of glycosylated exosomes, immunological detection of exosomes, or gene detection or analysis after extracting nucleic acids from the exosomes, and may also be subjected to a further purification so as to obtain exosomes with higher purity.

The above description shows and describes the preferred embodiments of the present invention. As previously mentioned, it should be understood that the present invention is not limited to the forms disclosed herein, should not be considered as excluding other embodiments and may be used for various other combinations, modifications and environments; and variations can be made through the above teachings or technologies or knowledges in the related art in the scope of the inventive concept herein. Any variations and changes made by persons of ordinary skill in the art without departing from the spirit and scope of the present invention should be inside the protection scope defined in the appended claims of the present invention.

### Industrial practicability

The present invention relates to a lectin-macromolecular carrier coupling complex for separating glycosylated exosomes from a clinical sample. The lectin-macromolecular carrier coupling complex comprises a macromolecular carrier and lectins coupled to the outer side of the macromolecular carrier. The lectin-macromolecular carrier coupling complex provided by the present invention may simply, conveniently, rapidly, and accurately separate glycosylated exosomes from a clinical sample with a high separation efficiency and a good repeatability; and the separated exosomes are intact in morphology without rupturing or cracking, and may be directly used for detection on glycosylated exosome liquid, or directly used for immunology-related detection, or directly used for gene detection or analysis after extracting nucleic acids from exosomes.

## Claims

1. A lectin-macromolecular carrier coupling complex for separating glycosylated exosomes from a clinical sample, comprising a macromolecular carrier and lectins coupled to the outer side of the macromolecular carrier, wherein
the lectins are any one type of Artocarpus integrifolia lectin, peanut lectin, Pisum sativum lectin (WA and/or VVL), Concanavalin lectin, Lens culinaris lectin, wheat germ lectin, soybean lectin, kidney bean lectin, and snail lectin (HAA and/or HPA), or a combination of two or more of the above; and
the macromolecular carrier is any one type of a dextran microsphere , an agarose microsphere, a resin or epoxy resin microsphere, and a polystyrene microsphere, or a combination of two or more of the above.

2. The lectin-macromolecular carrier coupling complex according to claim 1, wherein a particle size distribution range of the macromolecular carrier is from 1 µm to 200 µm, preferably, from 10 µm to 200 µm, and more preferably, from 30 µm to 150 µm.

3. The lectin-macromolecular carrier coupling complex according to claim 1, wherein 1-20 mg, preferably, 5-15 mg, and more preferably, 10-15 mg of the lectins are coupled to each 1 mL of the macromolecular carrier.

4. The lectin-macromolecular carrier coupling complex according to claim 1, wherein the clinical sample is any one of serum, plasma, saliva, a tissue or cell culture supernatant, urine, a cerebrospinal fluid, and a lymph fluid.

5. The lectin-macromolecular carrier coupling complex according to claim 1, wherein the lectins are any one type of Artocarpus integrifolia lectin, peanut lectin, Pisum sativum lectin (WA and/or VVL), Concanavalin lectin, Lens culinaris lectin, wheat germ lectin, soybean lectin, and kidney bean lectin, or a combination of two or more of the above.

6. The lectin-macromolecular carrier coupling complex according to claim 1, wherein the glycosylated exosomes are any one type of N-glycosylated exosomes, O-glycosylated exosomes, and fucosylated exosomes, or a combination of two or more of the above.

7. The lectin-macromolecular carrier coupling complex according to claim 1, wherein the lectin-macromolecular carrier coupling complex is preserved in a preservation solution, which is loaded into a separating device comprising an affinity adsorption centrifuge tube; the affinity adsorption centrifuge tube comprises two parts which are an upper centrifuge tube and an outer protective sleeve, respectively; the diameter of the upper centrifuge tube is smaller than that of the outer protective sleeve, the upper centrifuge tube is sleeved in the outer protective sleeve, and protruding annular edges or pillars are provided on the outer side of the upper part of the upper centrifuge tube and are used for supporting an opening of the upper centrifuge tube to be higher than the outer protective sleeve; the upper centrifuge tube comprises a centrifuge tube cap, a centrifuge tube wall, and a filter membrane bottom fixedly attached to the centrifuge tube wall, in which the filter membrane of the filter membrane bottom has a pore size smaller than the particle size of the lectin-macromolecular carrier coupling complex and larger than the particle sizes of exosomes, and the lectin-macromolecular carrier coupling complex preservation solution is retained in the upper centrifuge tube; and optionally, the pore size of the filter membrane is 150-1000 nm.

8. A composition for separating glycosylated exosomes, comprising the lectin-macromolecular carrier coupling complex according to any one of claims 1-7, a washing buffer for washing and for removing the exosomes nonspecifically bound to the coupling complex or not glycosylated and other impurities in the separation process, and/or an elution buffer for eluting the glycosylated exosomes specifically bound to the lectin-macromolecular carrier coupling complex.

9. The composition for separating glycosylated exosomes according to claim 8, wherein the washing buffer is a metal salt ion-free washing buffer or purified water, and optionally, is a metal salt ion-free washing buffer;
and/or the elution buffer is a borate buffer with saccharides dissolved therein, and further optionally, is a borate buffer with mannose dissolved therein.

10. A method of separating glycosylated exosomes, comprising a separation step of using the lectin-macromolecular carrier coupling complex according to any one of claims 1-7 to separate glycosylated exosomes, wherein the separation step comprises:
pretreatment of a clinical sample:
optionally diluting the pretreated sample by mixing the same with a washing buffer uniformly to obtain a sample to be detected;
completely removing the preservation solution portion in the lectin-macromolecular carrier coupling complex preservation solution, while retaining the lectin-macromolecular carrier coupling complex;
adding the sample to be detected to the lectin-macromolecular carrier coupling complex with removal of the preservation solution, leaving the mixture to stand still at room temperature for incubation, and removing the sample;
washing the lectin-macromolecular carrier coupling complex bound to the glycosylated exosomes with the washing buffer, and then removing the washing buffer for washing and for removing the exosomes nonspecifically bound to the coupling complex or not glycosylated and other impurities in the separation process; and
eluting the washed lectin-macromolecular carrier coupling complex with the elution buffer, leaving the system to stand still at room temperature, and collecting the elution supernatant, which is a solution of the separated glycosylated exosomes.

11. The method of separating glycosylated exosomes according to claim 10, wherein a ratio of the sample to be detected to the lectin-macromolecular carrier coupling complex is 1: (1-3);
and/or a ratio of the volume of the washing buffer in a single addition to the volume of the lectin-macromolecular carrier coupling complex bound to glycosylated exosomes is (1-3): 1; and/or the washing step with the washing buffer is repeated for 1-3 times;
and/or a ratio of the volume of the added elution buffer to the volume of the washed lectin-macromolecular carrier coupling complex is (0.5-2): 1, and preferably, is (0.5-1): 1.

12. The method of separating glycosylated exosomes according to claim 10, wherein completely removing the preservation solution portion in the lectin-macromolecular carrier coupling complex preservation solution, removing the sample, removing the washing buffer, and/or collecting the elution supernatant are all conducted by centrifugation at room temperature at a speed of 3000 rpm or below for 20 s,
and/or the incubation is conducted at room temperature for 10-30 min, and preferably, for 10-15 min;
and/or the washing solution is a metal salt ion-free washing buffer or purified water, and optionally, is a metal salt ion-free washing buffer;
and/or the elution buffer is a borate buffer with saccharides dissolved therein, and optionally, is a borate buffer containing mannose.

13. The method of separating glycosylated exosomes according to claim 10, wherein the pretreatment of a sample comprises the following steps:
for a serum sample, a plasma sample, a saliva sample, a cerebrospinal fluid sample, or a lymph fluid sample, subjecting the sample to centrifugation at a speed of 3000 g or below for 10-15 min,
to remove cell debris, precipitates, and other impurities in the sample, and taking the supernatant after centrifugation for later use; and
for a tissue or cell culture supernatant sample or a urine sample, subjecting the sample to centrifugation at a speed of 3000 g or below for 10-15 min, to remove cell debris, precipitates, and
other impurities in the sample, and then concentrating the supernatant by 10-1000 times through an ultrafiltration tube for later use.

14. The method of separating glycosylated exosomes according to claim 10, wherein the separation step of using the lectin-macromolecular carrier coupling complex according to claim 7 to separate the glycosylated exosomes comprises:
pretreatment of a clinical sample:
optionally diluting the pretreated sample by mixing the same with awashing buffer uniformly to obtain a sample to be detected;
completely removing the preservation solution portion in the lectin-macromolecular carrier coupling complex preservation solution from the affinity adsorption centrifuge tube loaded with the lectin-macromolecular carrier coupling complex, while retaining the lectin-macromolecular carrier coupling complex, and replacing the outer sleeve with a new one;
adding the sample to be detected to the upper centrifuge tube, from which the preservation solution has been removed, leaving the mixture to stand still at room temperature for incubation, and removing the sample;
adding the washing buffer to the upper centrifuge tube, taking out the upper centrifuge tube after centrifuging the affinity adsorption centrifuge tube, charging the upper centrifuge tube in a new outer sleeve, completely discarding the original outer sleeve and liquid therein, for washing and
for removing the exosomes nonspecifically bound to the coupling complex or not glycosylated and other impurities in the separation process, wherein this step is conducted for 1-3 times; and
adding the eluate to the upper centrifuge tube, to elute the washed lectin-macromolecular carrier coupling complex, leaving the system to stand still at room temperature, and collecting an elution supernatant, which is a solution of the separated glycosylated exosomes, from the outer sleeve.

15. Use of exosomes separated by using the method of separating glycosylated exosomes according to any one of claims 10-14, comprising uses for liquid detection of glycosylated exosomes, for immunological detection of the exosomes, or for detection or analysis of nucleotide fragments after nucleic acid extraction from the exosomes.
